# EUROPÄISCHE PATENTSCHRIFT

(11) **EP 3 352 713 B1**
(45) Veröffentlichungstag und Bekanntmachung des Hinweises auf die Patenterteilung: **30.01.2019**
(21) Anmeldenummer: 16774890.4
(22) Anmeldetag: 21.09.2016
(51) Int. Cl.: A61F 5/01

(54) **DYNAMISCHE GELENKSTÜTZE**
DYNAMIC ARTICULATION SUPPORT
SUPPORT DYNAMIQUE D'ARTICULATION

(30) Priorität: 23.09.2015 DE 102015012321
(43) Veröffentlichungstag der Anmeldung: 01.08.2018
(73) Patentinhaber: Albrecht GmbH, 83071 Stephanskirchen (DE)
(72) Erfinder: OPAHLE, Hans-Georg, 83024 Rosenheim (DE)
(74) Vertreter: Flach Bauer Stahl Patentanwälte Partnerschaft mbB
(86) Internationale Anmeldenummer: PCT/EP2016/072420
(87) Internationale Veröffentlichungsnummer: WO 2017/050824

(56) Entgegenhaltungen:
- EP-A1- 0 841 044
- WO-A1-01/58392
- WO-A1-03/070129
- WO-A1-2004/056293

## Beschreibung

Die Erfindung betrifft eine dynamische Gelenkstütze mit einer ersten und zweiten Schiene, die an Körpergliedern befestigbar und über ein Drehgelenk miteinander verbunden sind, gemäß dem Oberbegriff des Anspruches 1.

Insbesondere Gelenkkapseln und/oder Bindegewebe weisen beispielsweise nach Bänderoperationen, Unfällen, Entzündungen etc. häufig ein Streck- oder Beugedefizit auf. Dies bedeutet, dass ein distales Körperglied, beispielsweise ein Unterschenkel, nicht mehr vollständig in seine normale Extensions- oder Flexionslage bezüglich eines proximalen Körperglieds, beispielsweise eines Oberschenkels, gebracht werden kann.

Um einem derartigen Streck- oder Beugedefizit entgegenzuwirken, wird in bekannter Weise versucht, die Schrumpfungen wieder dadurch zu dehnen, dass das distale Körperglied bezüglich des proximalen Körperglieds mittels einer sogenannten Quengelvorrichtung in einer bestimmten Quengellage festgelegt oder in diese Quengellage mittels Federkraft vorgespannt wird.

Die Fixierung des Drehgelenkes in der Quengellage erfolgt bei bekannten Knieorthesen beispielsweise mittels Schrauben oder Stiften, welche in entsprechende Querbohrungen, die im Bereich des Drehgelenks der Knieorthese vorgesehen sind, eingeführt werden können. Nachteilig ist bei diesen bekannten Knieorthesen jedoch, dass der Patient bei fixiertem Drehgelenk nicht oder nur mit großen Schwierigkeiten gehen kann, da er das Knie nicht abbiegen kann. Darüber hinaus ist das Entfernen bzw. Einsetzen der Arretierstifte oder -schrauben mit einem gewissen Umstand verbunden. Von besonderer Bedeutung ist, dass die Fixierung von Ober- und Unterschenkel in der Quengellage häufig bereits nach kurzer Zeit Schmerzen verursacht, so dass die Quengellage nur kurze Zeit aufrechterhalten werden kann. Der Erfolg dieser Methode bezüglich des Ausgleichs eines Streck- oder Beugedefizits lässt daher stark zu wünschen übrig.

Aus der EP 0 841 044 A1 ist eine dynamische Gelenkstütze gemäß dem Oberbegriff des Anspruches 1 bekannt, welche die vorher erwähnten Nachteile wirkungsvoll vermeidet, zur Beseitigung von Streck- oder Beugedefiziten besonders geeignet ist und die darüber hinaus den Patienten ein besonders hohes Maß an Bewegungsmöglichkeit und Tragekomfort bietet. Dies wird dort durch eine eine Feder aufweisende Krafterzeugungseinrichtung, eine drehbar gelagerte Kupplungsscheibe, mittels der die Vorspannkraft der Feder auf die erste Schiene übertragbar ist, sowie durch einen Kraftblockiermechanismus erreicht, der von einem nichtblockierenden Zustand, in welchem die Kraft der Krafterzeugungseinrichtung über die Kupplungsscheibe frei auf die erste Schiene übertragbar ist, in einen blockierenden Zustand bringbar ist, in welchem die Kraft der Krafterzeugungseinrichtung von der ersten Schiene abgekoppelt ist und vom Kraftblockiermechanismus aufgenommen wird, so dass die erste Schiene auch frei ohne Federeinwirkung schwenkbar ist. Der Kraftblockiermechanismus besteht dort aus einem Stift, der in Axialrichtung der proximalen Schiene in das Drehgelenkgehäuse eingeschoben bzw. aus diesem herausgezogen wird, um die Drehbewegung der Kupplungsscheibe beim Beugen bzw. Strecken des Gelenks und damit die Übertragung der Kraft der Krafterzeugungseinrichtung auf die erste Schiene zu blockieren bzw. freizugeben.

Der Erfindung liegt die Aufgabe zugrunde, einen alternativen Kraftblockiermechanismus zu schaffen, der auf möglichst einfache Weise zu betätigen und bestmöglich in das Gehäuse des Drehgelenks integrierbar ist.

Diese Aufgabe wird erfindungsgemäß durch eine dynamische Gelenkstütze mit den Merkmalen des Anspruchs 1 gelöst. Vorteilhafte Ausführungsformen der Erfindung sind in den weiteren Ansprüchen beschrieben.

Bei der erfindungsgemäßen dynamischen Gelenkstütze umfasst der Kraftblockiermechanismus eine schwenkbar gelagerte, im Inneren des Gehäuses angeordnete Sperrklinke in der Form einer Wippe und eine manuell betätigbare Sperrklinkenbetätigungseinrichtung, mit welcher ein Endbereich der Wippe in- und außer Sperreingriff mit der Kupplungsscheibe bringbar ist.

Der erfindungsgemäße Kraftblockiermechanismus ist auf besonders einfache und sichere Weise zu betätigen und kann ohne weiteres so gestaltet werden, dass die Sperrklinkenbetätigungseinrichtung nur in geringem Maß über das Gehäuse vorsteht. Dadurch, dass die gegebenenfalls vom Kraftblockiermechanismus aufzunehmende Kraft nicht unmittelbar auf ein mit den Fingern zu bewegendes Betätigungselement einwirkt, sondern auf ein Zwischenelement in der Form einer Sperrklinke, wobei dann die mit den Fingern zu bewegende Sperrklinkenbetätigungseinrichtung lediglich die Sperrklinke zwischen einer Sperrstellung und Freigabestellung bewegen muss, ist die Betätigung des Kraftblockiermechanismus auf besonders einfache Weise möglich.

Gemäß einer vorteilhaften Ausführungsform ist die Sperrklinke um eine Schwenkachse schwenkbar, die parallel zu einer Schwenkachse des Drehgelenks angeordnet ist. Die Sperrklinke ist dabei durch die Schwenkachse der Sperrklinke in einen ersten und zweiten Sperrklinkenabschnitt unterteilt. Weiterhin umfasst die Sperrklinkenbetätigungseinrichtung eine relativ zum Gehäuse verschiebbare Schiebeeinrichtung, die wahlweise mit dem ersten oder zweiten Sperrklinkenabschnitt in Wirkverbindung bringbar ist, um das freie Ende des ersten Sperrklinkenabschnitts mittels einer Wippbewegung der Sperrklinke in und außer Sperreingriff mit der Kupplungsscheibe zu bringen. Auf diese Weise ist der Kraftblockiermechanismus mittels einer Schiebebewegung betätigbar, die mit einem einzigen Finger durchgeführt werden kann.

Vorteilhafterweise weist die Sperrklinkenbetätigungseinrichtung ein sich durch eine Wand des Gehäuses hindurch erstreckendes, relativ zum Gehäuse verschiebbares Handhabungsteil und eine am Handhabungsteil befestigte Federzunge auf, die wahlweise mit dem ersten oder zweiten Sperrklinkenabschnitt in Druckkontakt ist. Die Federzunge stellt ein elastisches Kopplungsglied zwischen dem Handhabungsteil und der Sperrklinke dar und kann sich, wenn das Handhabungsteil verschoben wird und die Federzunge auf der Sperrklinke entlang gleitet, dem Oberflächenverlauf der Sperrklinke optimal anpassen. Weiterhin ist es hierdurch möglich, durch das Verschieben des Handhabungsteils zunächst nur eine Druckvorspannung in Löserichtung aufzubringen, wobei die Sperrklinke dann erst zeitverzögert in die Lösestellung schwenkt, wenn sich die erste Schiene geringfügig gegenüber der zweiten Schiene bewegt und hierdurch die Reibkraft zwischen Sperrklinke und Kupplungsscheibe aufgehoben wird. Das Handhabungsteil lässt sich somit auch sehr einfache Weise verschieben.

Vorteilhafterweise hat das Gehäuse eine Schalenform mit einer Umfangswandung und einer Seitenwand. Die Sperrklinkenbetätigungseinrichtung ist dabei manuell längs der Umfangswand in Umfangsrichtung zwischen einer ersten Position, in welcher die Sperrklinkenbetätigungseinrichtung den ersten Sperrklinkenabschnitt beaufschlagt, und einer zweiten Position verschiebbar, in welcher die Sperrklinkenbetätigungseinrichtung den zweiten Sperrklinkenabschnitt beaufschlagt. Das Handhabungsteil der Sperrklinkenbetätigungseinrichtung lässt sich hierdurch auf sehr platzsparende und ergonomisch vorteilhafte Weise im Bereich der Umfangswand des Gehäuses anordnen.

Die Erfindung wird nachfolgend anhand der Zeichnungen beispielhaft näher erläutert. Es zeigen:
- Figur 1:: eine Seitenansicht der erfindungsgemäßen Gelenkstütze ohne Befestigungsgurte von einer ersten Seite her,
- Figur 2:: eine Seitenansicht der Gelenkstütze von Figur 1 von der entgegengesetzten Seite her,
- Figur 3:: eine Explosionsdarstellung der Gelenkstütze von Figur 1,
- Figur 4:: eine Seitenansicht der ersten Schiene in Alleinstellung,
- Figur 5:: eine Seitenansicht eines Winkelverstellelementes in Alleinstellung,
- Figur 6:: eine Seitenansicht einer Kupplungsscheibe in Alleinstellung,
- Figur 7:: eine räumliche Darstellung eines Schlittenelementes (Gleitschuhs) in Alleinstellung,
- Figur 8:: eine Seitenansicht des Gehäuses in Alleinstellung von einer ersten Seite her,
- Figur 9:: eine Seitenansicht des Gehäuses von Figur 8 von der entgegengesetzten Seite her,
- Figuren 10a bis 10d:: Ansichten bzw. einen Schnitt durch ein Vorspannungseinstellzahnrad,
- Figur 11:: eine Seitenansicht der Feder zur Erzeugung der Vorspannung zwischen distaler und proximaler Schiene,
- Figur 12:: eine Seitenansicht einer Zwischenscheibe zur Begrenzung des Drehbereichs des Vorspannungseinstellzahnrads,
- Figur 13:: eine Seitenansicht eines Handhabungsteils der Sperrklinkenbetätigungseinrichtung in Alleinstellung,
- Figur 14:: eine Seitenansicht einer Federzunge;
- Figur 15:: eine Seitenansicht einer Sperrklinke,
- Figur 16:: eine etwas schematisch dargestellte Seitenansicht des Gehäuses mit Kupplungsscheibe und Kraftblockiermechanismus, wobei sich der Kraftblockiermechanismus in einer Sperrstellung befindet, und
- Figur 17:: eine Seitenansicht entsprechend Figur 16, wobei sich der Kraftblockiermechanismus in der Freigabestellung befindet.

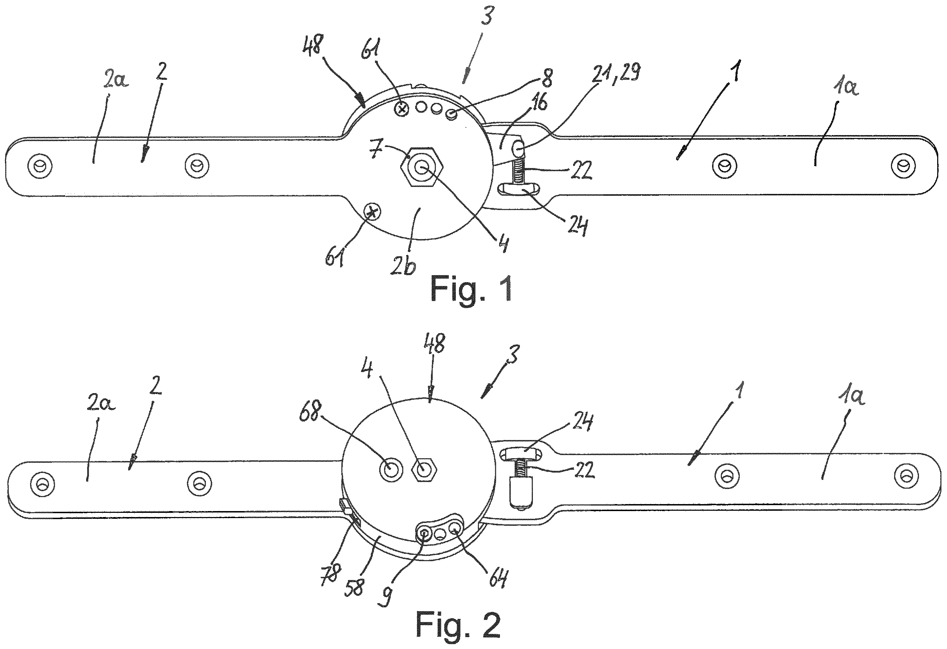

Im Folgenden wird anhand der Figuren 1 bis 17 eine erfindungsgemäße Gelenkstütze beschrieben, wobei im Normalfall zwei Gelenkstützen der dargestellten Art verwendet werden, die sich auf gegenüberliegenden Seiten des Gelenkes befinden. Die auf der gegenüberliegenden Seite des Gelenkes anzuordnende Gelenkstütze ist spiegelbildlich ausgebildet. Befestigungselemente wie zum Beispiel gepolsterte Schalen und Befestigungsgurte, die an der dargestellten Gelenkstütze befestigt werden und zur Befestigung der ersten und zweiten Schiene am distalen bzw. proximalen Körperglied dienen, sind der Übersichtlichkeit halber weggelassen. Weiterhin wird die erfindungsgemäße Gelenkstütze anhand des Beispiels einer Quengelvorrichtung für ein Knie beschrieben, könnte jedoch auch für andere Gelenke, beispielsweise für das Ellenbogengelenk, Anwendung finden.

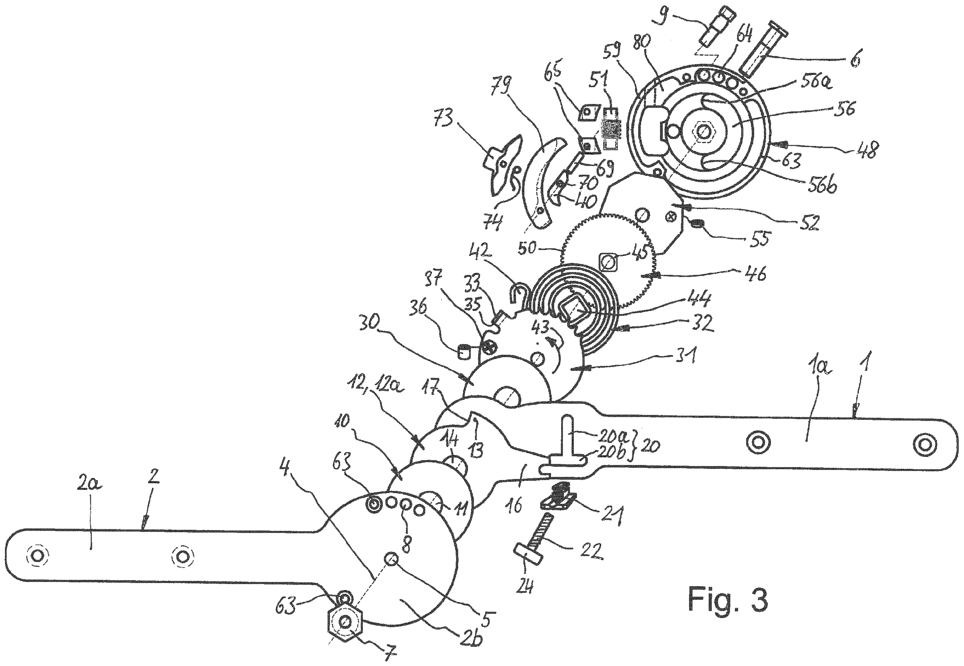

Wie aus Figur 1 und der Explosionsdarstellung von Figur 3 ersichtlich, weist die erfindungsgemäße Gelenkstütze eine erste Schiene 1 mit einem Befestigungsabschnitt 1a auf, welcher mittels entsprechender, nicht gezeigter Gurte beispielsweise am Unterschenkel befestigt werden kann, sowie eine zweite Schiene 2 mit einem Befestigungsabschnitt 2a, welcher ebenfalls mittels nicht gezeigter Gurte beispielsweise am Oberschenkel befestigbar und über ein Drehgelenk 3 gelenkig mit der ersten Schiene 1 verbunden ist. Die Schwenkachse ist hierbei mit dem Bezugszeichen 4 bezeichnet.

Wie aus Figur 3 ersichtlich, weist ein kreisscheibenförmiger Gelenkabschnitt 2b der zweiten Schiene 2 in seinem Zentrum ein Loch 5 zum Durchtritt eines Gelenkbolzens 6 auf, der mittels einer Mutter 7 festgelegt werden kann.

In der Nähe des Außenumfangs des Gelenkabschnittes 2b sind weiterhin drei Extensionsbegrenzungslöcher 8 vorgesehen, die in Umfangsrichtung nebeneinanderliegend angeordnet sind und zur Aufnahme eines Extensionsbegrenzungsstiftes 9 dienen. Zwei benachbarte Extensionsbegrenzungslöcher 8 sind in Umgangsrichtung jeweils mit einem Winkel von 15° versetzt angeordnet.

Die zweite Schiene 2 stellt das seitlich am weitesten innenliegende Teil der Gelenkstütze dar, das somit, mit einer entsprechenden Polsterung und/oder an den Oberschenkel angepassten Schalenteilen versehen, am Oberschenkel des Patienten anliegt.

Wie weiterhin aus Figur 3 ersichtlich, schließt an den Gelenkabschnitt 2b der zweiten Schiene 2 in Richtung nach außen eine kreisförmige Reibungsverminderungsscheibe 10 an, die beispielsweise aus einem dünnen Kunststoffblatt bestehen kann. Die Reibungsverminderungsscheibe 10 weist eine mittige Durchgangsöffnung 11 zum Durchtritt des Gelenkbolzens 6 auf.

In Richtung nach außen folgt ein scheibenförmiges Winkelverstellelement 12, das an der ersten Schiene 1 anliegt und um die Schwenkachse 4 herum relativ zur ersten Schiene 1 verschwenkt werden kann. Das Winkelverstellelement 12 umfasst einen halbkreisförmigen Abschnitt 12a, über den eine Anschlagnase 13 radial nach außen vorsteht. Eine mittige Durchgangsöffnung 14 im halbkreisförmigen Abschnitt 12a ermöglicht das Hindurchführen des Gelenkbolzens 6. Der Radius des halbkreisförmigen Abschnitts 12a ist kleiner als der Radius des Kreisbogens, auf dem die Extensionsbegrenzungslöcher 8 der zweiten Schiene 2 liegen. Die Extensionsbegrenzungslöcher 8 werden somit vom halbkreisförmigen Abschnitt 12a des Winkelverstellelementes 12 nicht abgedeckt.

An den halbkreisförmigen Abschnitt 12a des Winkelverstellelementes 12 schließt sich ein länglicher Handhabungsabschnitt 16 an, der einen Schwenkhebel zum Schwenken des Winkelverstellelementes 12 relativ zur ersten Schiene 1 bildet.

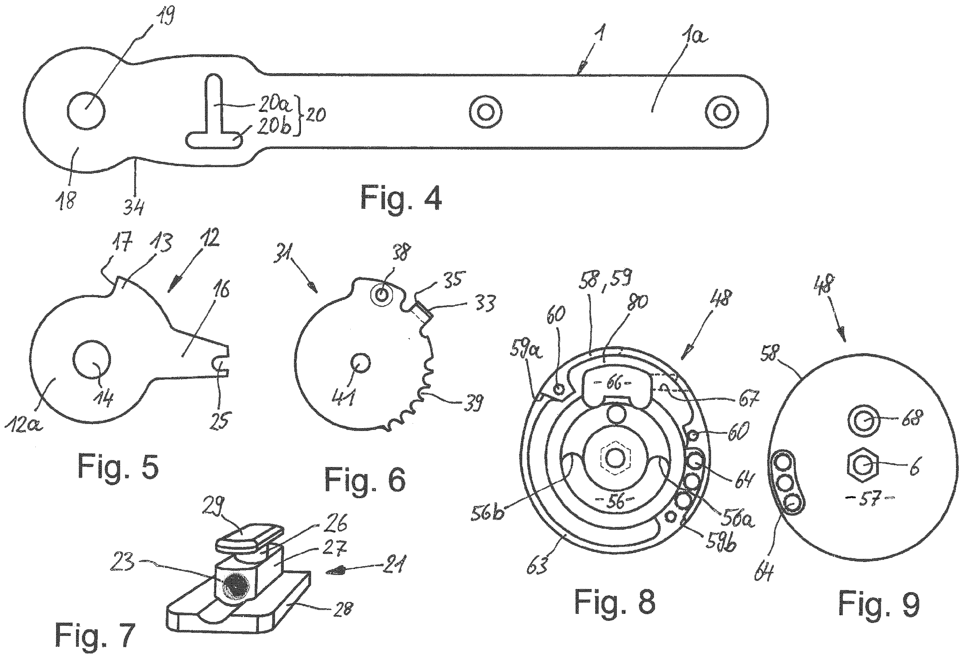

Die Anschlagnase 13 des Winkelverstellelementes 12 erstreckt sich in radialer Richtung über die Extensionsbegrenzungslöcher 8 hinaus nach außen, so dass eine stirnseitige Kontaktfläche 17 (Figur 5) der Anschlagnase 13 an dem Extensionsbegrenzungsstift 9 anschlagen kann, der in eines der Extensionsbegrenzunslöcher 8 der zweiten Schiene 2 eingeführt ist.

Wie weiterhin aus Figur 4 ersichtlich, weist die erste Schiene 1 in ihrem gelenknahen Endbereich 18 eine Durchgangsöffnung 19 zum Hindurchführen des Gelenkbolzens 6 auf. Dieser Endbereich 18 ist überwiegend kreisförmig ausgebildet, wobei sich der Kreisbogen über etwas mehr als 180°, beispielsweise 220°, erstrecken kann. Der Radius des Endbereiches 18 ist gleich groß oder etwas kleiner als der Radius des etwa halbkreisförmigen Abschnitts 12a des Winkelverstellelementes 12.

Wie aus den Figuren 3 und 4 ersichtlich, weist die erste Schiene 1 weiterhin eine T-förmige Aussparung 20 auf. Die Aussparung 20 umfasst einen Querabschnitt 20a, der rechtwinklig zur Längsachse der ersten Schiene 1 verläuft, sowie einen kürzeren Längsabschnitt 20b, der parallel zur Längsachse der ersten Schiene 1 verläuft. Der Querabschnitt 20a dient zur Schiebeführung eines Schlittenelementes 21 (Gleitschuhs), das in Figur 7 näher dargestellt ist. Das Schlittenelement 21 kann nach Art eines Nutsteins längs des Querabschnitts 20a der Aussparung 20 verschoben werden. Das Verschieben erfolgt mittels einer Stellschraube 22 (Figur 3), die in eine Gewindebohrung 23 (Figur 7) des Schlittenelementes 21 eingeschraubt werden kann. Der Kopf 24 der Stellschraube 22 ist dabei im Längsabschnitt 20b der Aussparung 20 festgelegt. Durch manuelles Drehen der Stellschraube 22 kann damit das Schlittenelement 21 spindelartig quer zur distalen Schiene 1 verschoben werden.

Der Handhabungsabschnitt 16 des Winkelverstellelementes 12 weist an seinem freien Ende eine Aussparung 25 (Figur 5) auf, in die ein Kupplungsbolzen 26 (Figur 7) des Schlittenelementes 21 formschlüssig eingreift. Wird das Schlittenelement 21 mittels der Stellschraube 22 quer zur ersten Schiene 1 verschoben, wird somit der Handhabungsabschnitt 16 des Winkelverstellelementes 12 mit bewegt und das Winkelverstellelement 12 um die Schwenkachse 4 herum relativ zur ersten Schiene 1 verschwenkt. Hierdurch nimmt die Anschlagnase 13 unterschiedliche Winkelpositionen relativ zur ersten Schiene 1 ein, wodurch der Schwenkbereich der ersten Schiene 1 zusätzlich zu den durch die Extensionsbegrenzungslöcher 8 bzw. den darin eingesteckten Extensionsbegrenzungsstift 9 vorgegebenen 15°-Schritten stufenlos verstellt werden kann.

Ein quaderförmiges Zwischenstück 27 des Schlittenelementes 21 (Figur 7), in dem die Gewindebohrung 23 vorgesehen ist, ist innerhalb des Querabschnittes 20a der T-förmigen Aussparung 20 angeordnet und geführt, während eine vergrößerte Grundplatte 28 die erste Schiene 1 untergreift und ein im Vergleich zum Kupplungsbolzen 26 verbreiteter Kopf 29 das Abheben des Winkelverstellelementes 12 vom Kupplungsbolzen 26 verhindert.

An der Außenseite der ersten Schiene 1 schließt sich eine weitere Reibungsverminderungsscheibe 30 an, die in gleicher Weise wie die Reibungsverminderungsscheibe 10 ausgebildet ist.

An die Reibungsverminderungsscheibe 30 schließt sich, wie aus Figur 3 ersichtlich, eine scheibenförmige Kupplungsscheibe 31 an, die dazu dient, die Federkraft einer axial nach außen benachbarten Feder 32 auf die erste Schiene 1 zu übertragen. Hierzu weist die Kupplungsscheibe 31, wie näher aus Figur 6 ersichtlich, in ihrem Randbereich eine Mitnehmerlasche 33 auf, die in axialer Richtung über die Grundfläche der Kupplungsscheibe 31 in Richtung zur ersten Schiene 1 vorsteht und den seitlichen Rand der distalen Schiene 1 an einer Kontaktstelle 34 (Figur 4) untergreift. Diejenige Kontaktstelle an der Kupplungsscheibe 31, die mit der Kontaktstelle 34 der ersten Schiene 1 in Druckkontakt gelangt, ist in Figur 6 mit dem Bezugszeichen 35 versehen.

Auf der entgegengesetzten Seite ist im Randbereich der Kupplungsscheibe 31 ein Mitnehmerbolzen 36 befestigt (Figur 3). Dieser Mitnehmerbolzen 36 steht über die Grundfläche der Kupplungsscheibe 31 in Richtung der Feder 32 vor. Die Befestigung des Mitnehmerbolzens 36 kann mittels einer Schraube 37 erfolgen, die durch eine Bohrung 38 (Figur 6) der Kupplungsscheibe 31 hindurchgeführt wird.

Die Kupplungsscheibe 31 weist ferner, wie aus Figur 6 ersichtlich, in ihrem Randbereich eine Mehrzahl von nebeneinanderliegenden, sich radial nach innen erstreckenden Aussparungen auf, die eine Verzahnung 39 bilden. In diesem Umfangsabschnitt ist die Kupplungsscheibe 31 somit ähnlich wie ein Zahnrad mit Stirnverzahnung ausgebildet. Diese Verzahnung 39 wirkt, wie später noch näher erläutert wird, mit einer Sperrklinke 40 zusammen, um die Drehbewegung der Kupplungsscheibe 31 um die Schwenkachse 4 herum bei Bedarf sperren zu können und dadurch die Kraft der Feder 32 von der ersten Schiene 1 abzukoppeln.

Die Kupplungsscheibe 31 weist wiederum ein mittiges Durchgangsloch 41 zum Hindurchführen des Gelenkbolzens 6 auf.

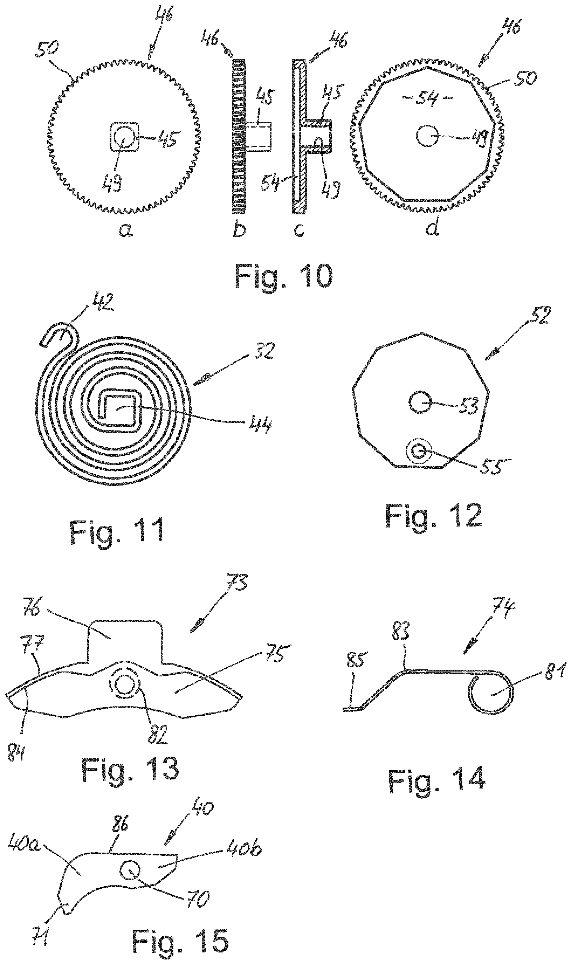

Wie aus Figur 3 weiterhin ersichtlich, schließt sich die Feder 32, die als Spiralfeder ausgebildet ist, seitlich an die Kupplungsscheibe 31 an. Die Feder 32 stellt eine Krafterzeugungseinrichtung zur Erzeugung einer Kraft in einer bestimmten Drehrichtung dar. Das außenliegende freie Ende der Feder 32 ist, wie auch aus Figur 11 ersichtlich, als Kopplungsauge 42 ausgebildet. In das Kopplungsauge 42 ist der an der Kupplungsscheibe 31 befestigte Mitnehmerbolzen 36 einführbar. Im zusammengebauten Zustand übt die Feder 32 über den Mitnehmerbolzen 36 eine permanente Vorspannkraft auf die Kupplungsscheibe 31 aus, welche diese in Richtung des Pfeiles 43 (Figur 3) um die Schwenkachse 4 herum zu drehen versucht. Das innenliegende Ende der Feder 32 ist in der Form eines mittigen viereckigen Durchgangslochs 44 gewickelt, so dass die Feder 32 auf einen entsprechend viereckigen mittigen Haltezapfen 45 eines benachbarten Vorspannungseinstellzahnrads 46 (siehe auch Figur 10) aufgeschoben werden kann. Durch Veränderung der Drehstellung des Vorspannungseinstellzahnrads 46 relativ zu einem Gehäuse 48 und damit relativ zur zweiten Schiene 2, die fest mit dem Gehäuse 48 verbunden ist, lässt sich somit die Kraft der Feder 32 einstellen, welche die erste Schiene 1 um die Schwenkachse 4 herum zu schwenken versucht.

Wie aus Figur 10 ersichtlich, weist das Vorspannungseinstellzahnrad 46 eine mittige Durchgangsöffnung 49 zur drehbaren Lagerung am darin durchgeführten Gelenkbolzen 6 auf. Weiterhin ist das Vorspannungseinstellzahnrad 46 als Schneckenrad ausgebildet, das mittels einer Stirnverzahnung 50 mit einer Schnecke 51 (Figur 3) zusammenwirkt. Die Schnecke 51 ist in einem Randbereich des Gehäuses 48 drehbar, jedoch axial unverschiebbar gelagert und kann durch eine Bohrung 67 (Figur 8) in der Wand des Gehäuses 48 hindurch mittels eines geeigneten Werkzeugs gedreht werden, um bei Bedarf die Drehposition des Vorspannungseinstellzahnrads 46 innerhalb des Gehäuses 48 zu verändern.

Damit die Drehposition des Vorspannungseinstellzahnrads 46 nur innerhalb eines bestimmten Drehbereichs möglich ist und die Feder 32 nicht zu stark auf- bzw. abgewickelt werden kann, wirkt das Vorspannungseinstellzahnrad 46 mit einer Drehbegrenzungsscheibe 52 zusammen, die in den Figuren 3 und 12 dargestellt ist. Diese Drehbegrenzungsscheibe 52 ist drehfest mit dem Vorspannungseinstellzahnrad 46 gekoppelt und weist eine mittige Durchgangsöffnung 53 zur drehbaren Lagerung am Gelenkbolzen 6 auf. Zur Drehkopplung mit dem Vorspannungseinstellzahnrad 46 ist der äußere Rand der Drehbegrenzungsscheibe 52 mehreckig ausgebildet. Das Vorspannungseinstellzahnrad 46 weist, wie aus Figur 10c, 10d ersichtlich, eine Vertiefung 54 mit einer entsprechend mehreckigen Außenkontur auf, so dass die Drehbegrenzungsscheibe 52 drehfest in die Vertiefung 54 eingesetzt werden kann. Zweckmäßigerweise weist die Vertiefung 54 eine Tiefe auf, die der Dicke der Drehbegrenzungsscheibe 52 entspricht, so dass die Drehbegrenzungsscheibe 52 axial nicht über das Vorspannungseinstellzahnrad 46 vorragt.

Auf der dem Vorspannungseinstellzahnrad 46 abgewandten Seite weist die Drehbegrenzungsscheibe 52 einen Drehbegrenzungsanschlag 55 in der Form eines axial vorstehenden Bolzens auf. Dieser Drehbegrenzungsanschlag 55 greift in eine vertiefte Nut 56 (Figur 8) des Gehäuses 48 ein, die sich kreisbogenförmig über einen Winkelbereich von etwa 180° um die Schwenkachse 4 herum erstreckt und Enden 56a, 56b aufweist, an denen der Drehbegrenzungsanschlag 55 bei Erreichen der Drehbereichsgrenze anschlägt.

Wie aus den Figuren 1-3, 8, 9 ersichtlich, ist das Gehäuse 48 schalen- bzw. napfförmig ausgebildet und weist somit eine Seitenwand 57 auf, über die eine Umfangswand 58 in axialer Richtung vorragt. Ein in Figur 8 näher dargestellter Abschnitt 59 der Umfangswand 58, der einerseits bei 59a und andererseits bei 59b begrenzt ist und sich über etwa 180° erstreckt, steht am weitesten axial über die Seitenwand 57 vor und stellt denjenigen Abschnitt dar, der auf die zweite Schiene 2 aufgesetzt wird. Gewindebohrungen 60 dienen zum Einschrauben von Befestigungsschrauben 61 (Figur 1), die durch Bohrungen 62 (Figur 3) der zweiten Schiene 2 hindurchgeführt werden.

Ein weiterer Abschnitt 63 der Umfangswand 58 steht axial weniger weit über die Seitenwand 57 des Gehäuses 48 vor, so dass ein Spalt geschaffen wird, durch den die erste Schiene 1 hindurchtreten kann.

Weiterhin weist das Gehäuse 48 Extensionsbegrenzungslöcher 64 auf, die zu den Extensionsbegrenzungslöchern 8 der zweiten Schiene 2 fluchten und zum Einstecken des Extensionsbegrenzungsstifts 9 in unterschiedlichen Winkelpositionen dienen.

Die Schnecke 51 ist mit ihren Endbereichen in Lagerstücken 65 (Figur 3) drehbar gelagert, die formschlüssig in einer Tasche 66 des Gehäuses 48 festgelegt ist. In Figur 8 ist mit gestrichelten Linien die seitliche Bohrung 67 angedeutet, durch die hindurch das Werkzeug zum Drehen der Schnecke 51 eingeführt werden kann. In Figur 9 ist weiterhin ein Schauloch 68 in der Seitenwand 57 dargestellt, durch das hindurch Markierungen sichtbar sind, die auf der Drehbegrenzungsscheibe 52 angeordnet sind, so dass die Drehposition der Drehbegrenzungsscheibe 52 und damit des Vorspannungseinstellzahnrads 46 abgelesen werden kann.

Um die Vorspannkraft der Feder 32 bei Bedarf von der ersten Schiene 1 abkoppeln zu können und ein freies Schwenken der ersten Schiene 1 relativ zur proximalen Schiene 2 ohne Kraftbeaufschlagung zu ermöglichen, ist weiterhin ein Kraftblockiermechanismus vorgesehen, der von einem nichtblockierenden Zustand, in welchem die Kraft der Feder 32 über die Kupplungsscheibe 31 frei auf die erste Schiene 1 übertragbar ist, in einen blockierenden Zustand bringbar ist, in welchem die Kraft der Feder 32 von der distalen Schiene 1 abgekoppelt ist und vom Kraftblockiermechanismus aufgenommen wird.

Dieser Kraftblockiermechanismus umfasst die bereits erwähnte Sperrklinke 40, die im Inneren des Gehäuses 48 angeordnet und in Form einer Wippe ausgebildet ist. Die Sperrklinke 40 ist auf einem Lagerstift 69 (Figur 3) schwenkbar gelagert, der eine Schwenkachse bildet, die sich parallel zur Schwenkachse 4 erstreckt. Der Lagerstift 69 ist auf einem der Lagerstücke 65 befestigt und steht über dieses Lagerstück 65 vor. Die Sperrklinke 40 weist hierzu zwischen ihren Enden eine entsprechende Lagerbohrung 70 auf, durch die sich der Lagerstift 69 hindurcherstreckt. Auf der einen Seite der Schwenkachse 87 weist die Sperrklinke somit einen ersten Sperrklinkenabschnitt 40a und auf der anderen Seite einen zweiten Sperrklinkenabschnitt 40b auf.

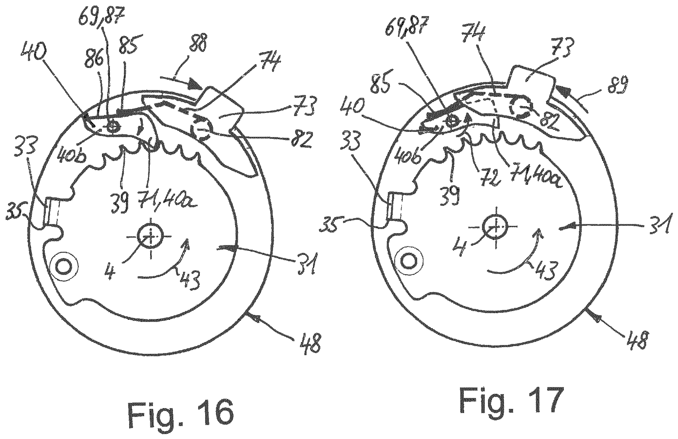

Wie aus den Figuren 16 und 17 ersichtlich, ist die Sperrklinke 40 derart in der Nähe des Randbereichs des Gehäuses 48 und radial außerhalb der Kupplungsscheibe 31 angeordnet, dass ein freier Endbereich 71 des ersten Sperrklinkenabschnitts 40a wahlweise in- und außer Eingriff mit der Verzahnung 39 der Kupplungsscheibe 31 gebracht werden kann. Befindet sich die Sperrklinke 40, wie in Figur 16 dargestellt, in Sperreingriff mit der Verzahnung 39, ist eine Drehbewegung der Kupplungsscheibe 31 in Richtung des Pfeils 43 gesperrt. Die in die Kupplungsscheibe 31 eingeleitete Vorspannkraft der Feder 32 wird über die Sperrklinke 40 auf den Lagerstift 69 und von diesem über das Lagerstück 65 in das Gehäuse 48 eingeleitet. Die erste Schiene 1 kann ohne Kraftbeaufschlagung in Richtung des Pfeils 43 von der Mitnehmerlasche 33 entfernt werden, das heißt die erste Schiene 1 ist ohne Kraftbeaufschlagung frei schwenkbar.

Wird dagegen, wie aus Figur 17 ersichtlich, die Sperrklinke 40 derart in Richtung des Pfeils 72 hoch geschwenkt, dass sie außer Eingriff mit der Verzahnung 39 gelangt, wird die Kupplungsscheibe 31 ungehindert durch die Kraft in Drehrichtung, das heißt in Richtung des Pfeils 43, gedrängt, so dass über die Mitnehmerlasche 33 eine entsprechende Schwenkkraft auf die erste Schiene 1 ausgeübt wird.

Um die Sperrklinke 40 von der in Figur 16 dargestellten Sperrposition in die in Figur 17 dargestellte Freigabeposition zu schwenken, ist ein Handhabungsteil 73 (Figur 13) und eine am Handhabungsteil 73 befestigte Federzunge 74 (Figur 14) vorgesehen. Das Handhabungsteil 73 umfasst ein im Inneren des Gehäuses 48 angeordnetes Lagerteil 75, über das ein Schiebeknopf 76 radial nach außen vorsteht. Das Lagerteil 75 weist beidseitig des Schiebeknopfs 76 eine kreisbogenförmige Außenkontur 77 auf, die an der Innenseite der Umfangswand 58 des Gehäuses 48 anliegt und an dieser in Umfangsrichtung entlanggleiten kann. Der Schiebeknopf 76 erstreckt sich durch eine längliche Aussparung 78 (Figur 2) der Umfangswand 58 hindurch nach außen, so dass er mit einem Finger relativ zum Gehäuse 48 in dessen Umfangsrichtung verschoben werden kann.

Um das Handhabungsteil 73 an der Umfangswand 58 des Gehäuses 48 zu halten, ist weiterhin ein in Figur 3 dargestelltes bogenförmiges Füllstück 79 vorgesehen, das formschlüssig in eine Tasche 80 des Gehäuses 48 eingesetzt wird und den Schieberaum, in dem das Handhabungsteil 73 verschoben werden kann, radial nach innen begrenzt.

Die in den Figuren 14, 16 und 17 näher dargestellte Federzunge 74 ist in einem Endbereich 78 am Handhabungsteil 73 befestigt. Hierzu weist das Handhabungsteil 73 einen Zapfen 82 auf, um den der Endbereich 81 der Federzunge 74 herum geführt ist. Weiterhin weist die Federzunge 74 einen abgewinkelten Knieabschnitt 83 auf, mit dem sich die Federzunge 74 an einer Wand 84 des Lagerteils 75 (Figur 13) abstützen kann, so dass die Federzunge 74 daran gehindert wird, in diesem Kniebereich 83 radial nach außen auszuwandern, wenn ihr freies Ende 85 radial nach außen druckbeaufschlagt wird.

Wie aus den Figuren 16 und 17 ersichtlich, liegt das freie Ende 85 der Federzunge 74 an der radial äußeren Fläche 86 der Sperrklinke 40 auf. Die Federzunge 74 ist dabei derart vorgespannt, dass das freie Ende 85 permanent eine Druckkraft radial nach innen auf die Sperrklinke 40 ausübt. Drückt bei der in Figur 16 dargestellten Anordnung die Federzunge 74 auf den ersten Sperrklinkenabschnitt 40a, der rechts von der durch den Lagerstift 69 gebildeten Schwenkachse 87 der Sperrklinke 40 liegt, wird der Endbereich 71 der Sperrklinke 40 durch die Federzunge 74 in die Verzahnung 39 der Kupplungsscheibe 31 gedrückt und damit in Sperreingriff gebracht. Die Kupplungsscheibe 31 kann sich in dieser Sperrposition nicht mehr in Richtung des Pfeils 43 drehen. Die Kraft der Feder 32 ist somit, wie bereits ausgeführt, von der ersten Schiene 1 abgekoppelt.

Die Federzunge 74 nimmt die in Figur 16 dargestellte Stellung dann ein, wenn das Handhabungsteil 73 in Richtung des Pfeils 88, das heißt in Figur 16 nach rechts bzw. im Uhrzeigersinn, verschoben worden ist. Diese Position stellt eine erste Position dar.

Wird dagegen, wie aus Figur 17 ersichtlich, das Handhabungsteil 73 in Richtung des Pfeils 89 nach links bzw. entgegen dem Uhrzeigersinn in Richtung einer zweiten Position verschoben, verschiebt sich auch das freie Ende 85 der Federzunge 74 derart, dass es nunmehr eine radial nach innen gerichtete Druckkraft auf den zweiten Sperrklinkenabschnitt 40b ausübt, der auf der entgegengesetzten, das heißt linken Seite der Schwenkachse 87 liegt. Hierdurch wird der Endbereich 71 angehoben und außer Eingriff mit der Verzahnung 39 gebracht. Die Kupplungsscheibe 31 kann sich nunmehr frei in Richtung des Pfeils 43 drehen. Die Vorspannkraft der Feder 32 kann dadurch über die Kupplungsscheibe 31 auf die erste Schiene 1 übertragen werden.

## Patentansprüche

1. Dynamische Gelenkstütze mit
- einer ersten und zweiten Schiene (1, 2), die an Körpergliedern befestigbar und über ein Drehgelenk (3) miteinander verbunden sind,
- einer Krafterzeugungseinrichtung, die in einem unblockierten Zustand auf die erste Schiene (1) eine Kraft in einer bestimmten Drehrichtung ausübt,
- einer drehbar gelagerten Kupplungsscheibe (31), mittels der die Kraft der Krafterzeugungseinrichtung auf die erste Schiene (1) übertragbar ist,
- einem im Bereich des Drehgelenks (3) angeordneten Gehäuse (48), in dem die Krafterzeugungseinrichtung und Kupplungsscheibe (31) angeordnet sind,
- einem Kraftblockiermechanismus, der von einem nicht blockierenden Zustand, in welchem die Kraft der Krafterzeugungseinrichtung über die Kupplungsscheibe (31) frei auf die erste Schiene (1) übertragbar ist, in einen blockierenden Zustand bringbar ist, in welchem die Kraft der Krafterzeugungseinrichtung von der ersten Schiene (1) abgekoppelt ist und vom Kraftblockiermechanismus aufgenommen wird, so dass die erste Schiene (1) ohne Krafteinwirkung schwenkbar ist,
**dadurch gekennzeichnet, dass** der Kraftblockiermechanismus eine schwenkbar gelagerte, im Inneren des Gehäuses (48) angeordnete Sperrklinke (40) in der Form einer Wippe und eine manuell betätigbare Sperrklinkenbetätigungseinrichtung (73, 74) umfasst, mit welcher ein Endbereich (71) der Wippe in und außer Sperreingriff mit der Kupplungsscheibe (31) bringbar ist.

2. Gelenkstütze nach Anspruch 1, **dadurch gekennzeichnet, dass** die Sperrklinke (40) um eine Schwenkachse (87) schwenkbar ist, die parallel zu einer Schwenkachse (4) des Drehgelenks (3) angeordnet ist, dass die Sperrklinke (40) durch die Schwenkachse (87) in einen ersten und zweiten Sperrklinkenabschnitt (40a, 40b) unterteilt ist, und dass die Sperrklinkenbetätigungseinrichtung (73, 74) eine relativ zum Gehäuse (48) verschiebbare Schiebeeinrichtung umfasst, die wahlweise mit dem ersten oder zweiten Sperrklinkenabschnitt (40a, 40b) in Wirkeingriff bringbar ist, um den ersten Sperrklinkenabschnitt (40a) mittels einer Wippbewegung der Sperrklinke (40) in- und außer Sperreingriff mit der Kupplungsscheibe (31) zu bringen.

3. Gelenkstütze nach Anspruch 2, **dadurch gekennzeichnet, dass** die Sperrklinkenbetätigungseinrichtung (73, 74) ein relativ zum Gehäuse (48) verschiebbares Handhabungsteil (73) und eine am Handhabungsteil (73) befestigte Federzunge (74) aufweist, die wahlweise mit dem ersten oder zweiten Sperrklinkenabschnitt (40a, 40b) in Druckkontakt ist.

4. Gelenkstütze nach Anspruch 2 oder 3, **dadurch gekennzeichnet, dass** das Gehäuse (48) eine Schalenform hat und eine Umfangswand (58) und eine Seitenwand (57) aufweist, und dass die Sperrklinkenbetätigungseinrichtung (73, 74) manuell längs der Umfangswand (58) zwischen einer ersten Position, in welcher die Sperrklinkenbetätigungseinrichtung (73, 74) den ersten Sperrklinkenabschnitt (40a) beaufschlagt, und einer zweiten Position verschiebbar ist, in welcher die Sperrklinkenbetätigungseinrichtung (73, 74) den zweiten Sperrklinkenabschnitt (40b) beaufschlagt.

## Claims

1. Dynamic joint brace comprising
- a first and second bar (1, 2) which are attachable to limbs and interconnected by means of a rotational joint (3),
- a force-generating means which, in an unblocked state, exerts a force upon the first bar (1) in a determined direction of rotation,
- a rotatably mounted coupling disc (31), by means of which the force of the force-generating means can be transferred to the first bar (1),
- a housing (48) that is arranged in the region of the rotational joint (3) and in which the force-generating means and coupling disc (31) are arranged,
- a force-blocking mechanism which can be brought from a non-blocking state, in which the force of the force-generating means can be freely transferred to the first bar (1) by means of the coupling disc (31), into a blocking state, in which the force of the force-generating means is decoupled from the first bar (1) and absorbed by the force-blocking mechanism, such that the first bar (1) is pivotable without force being applied, **characterised in that** the force-blocking mechanism comprises a pivotably mounted pawl (40) in the form of a rocker that is arranged in the interior of the housing (48) and a manually operable pawl-operating means (73, 74), by means of which an end region (71) of the rocker can be brought into and out of latching engagement with the coupling disc (31).

2. Joint brace according to claim 1, **characterised in that** the pawl (40) is pivotable about a pivot axis (87) which is arranged in parallel with a pivot axis (4) of the rotational joint (3), **in that** the pawl (40) is divided into a first and second pawl portion (40a, 40b) by the pivot axis (87), and **in that** the pawl-operating means (73, 74) comprises a sliding means that can be moved relative to the housing (48) and can be brought into operative engagement with either the first or second pawl portion (40a, 40b), in order to bring the first pawl portion (40a) into and out of latching engagement with the coupling disc (31) by means of a rocking movement of the pawl (40).

3. Joint brace according to claim 2, **characterised in that** the pawl-operating means (73, 74) has a handling part (73) that can be moved relative to the housing (48) and a spring tab (74) that is attached to the handling part (73) and is in pressure contact with either the first or second pawl portion (40a, 40b).

4. Joint brace according to either claim 2 or claim 3, **characterised in that** the housing (48) is cup-shaped and comprises a peripheral wall (58) and a side wall (57), and **in that** the pawl-operating means (73, 74) can be moved manually along the peripheral wall (58) between a first position, in which the pawl-operating means (73, 74) acts on the first pawl portion (40a), and a second position, in which the pawl-operating means (73, 74) acts on the second pawl portion (40b).

## Revendications

1. Support dynamique pour articulation, comportant
- une première et une seconde tringle (1, 2) qui sont susceptibles d'être fixées à des membres du corps et qui sont reliées l'une à l'autre par une articulation rotative (3),
- un moyen générateur de force qui, dans un état non bloqué, exerce une force sur la première tringle (1) dans une direction de rotation déterminée,
- un disque d'accouplement (31) monté mobile en rotation, permettant de transmettre la force du moyen générateur de force à la première tringle (1),
- un boîtier (48) agencé dans la zone de l'articulation rotative (3), dans lequel sont agencés le moyen générateur de force et le disque d'accouplement (31),
- un mécanisme de blocage de force qui est susceptible d'être amené depuis un état non bloqué dans lequel la force du moyen générateur de force est librement transférable à la première tringle (1) par l'intermédiaire du disque d'accouplement (31), jusque dans un état bloqué dans lequel la force du moyen générateur de force est découplée de la première tringle (1) et est encaissée par le mécanisme de blocage de force, de sorte que la première tringle (1) est mobile en pivotement sans appliquer de force,
**caractérisé en ce que** le mécanisme de blocage de force comprend un cliquet d'arrêt (40) sous la forme d'une bascule agencée à l'intérieur du boîtier (48) et un moyen d'actionnement de cliquet d'arrêt (73, 74) à actionnement manuel permettant d'amener une zone d'extrémité (71) de la bascule en engagement d'arrêt avec le disque d'accouplement (31) et en dégagement de celui-ci.

2. Support pour articulation selon la revendication 1,
**caractérisé en ce que**
le cliquet d'arrêt (40) est mobile en pivotement autour d'un axe de pivotement (7) qui est agencé parallèlement à un axe de pivotement (4) de l'articulation rotative (3), **en ce que**
le cliquet d'arrêt (40) est subdivisé par l'axe de pivotement (87) en une première et en une seconde portion de cliquet d'arrêt (40a, 40b), et **en ce que**
le moyen d'actionnement de cliquet d'arrêt (73, 74) comprend un moyen de translation mobile par rapport au boîtier (48) et susceptible d'être amené en engagement actif sélectivement avec la première ou avec la seconde portion de cliquet d'arrêt (40a, 40b), en vue d'amener la première portion de cliquet d'arrêt (40a) en engagement d'arrêt avec le disque d'accouplement (31) et en dégagement de celui-ci par l'intermédiaire d'un mouvement de basculement du cliquet d'arrêt (40).

3. Support pour articulation selon la revendication 2,
**caractérisé en ce que**
le moyen d'actionnement de cliquet d'arrêt (73, 74) comprend un élément formant manette (73) mobile par rapport au boîtier (48), et une languette élastique (74) qui est fixée à l'élément formant manette (73) et qui est en contact de pression avec la première ou avec la seconde portion de cliquet d'arrêt (40a, 40b).

4. Support pour articulation selon la revendication 2 ou 3,
**caractérisé en ce que**
le boîtier (48) présente une forme de coque et comprend une paroi périphérique (58) et une paroi latérale (57), et **en ce que**
le moyen d'actionnement de cliquet d'arrêt (73, 74) est mobile manuellement le long de la paroi périphérique (58) entre une première position dans laquelle le moyen d'actionnement de cliquet d'arrêt (73, 74) sollicite la première portion de cliquet d'arrêt (40a), et une seconde position dans laquelle le moyen d'actionnement de cliquet d'arrêt (73, 74) sollicite la seconde portion de cliquet d'arrêt (40b).
